# EUROPEAN PATENT SPECIFICATION

(11) **EP 2 693 964 B1**
(45) Date of publication and mention of the grant of the patent: **19.06.2019**
(21) Application number: 12763492.1
(22) Date of filing: 02.04.2012
(51) Int. Cl.: A61B 17/70, A61B 17/86

(54) **POSTERIOR CERVICAL STABILIZATION SYSTEM**
SYSTEM ZUR STABILISIERUNG EINER NACH HINTEN GEKNICKTEN GEBÄRMUTTER
SYSTÈME DE STABILISATION CERVICALE POSTÉRIEURE

(30) Priority: 01.04.2011 US 201161470885 P
(43) Date of publication of application: 12.02.2014
(73) Proprietor: Stachniak, Rebecca Elizabeth, Plano, TX 75093 (US)
(72) Inventor: Stachniak, Rebecca Elizabeth, Plano, TX 75093 (US)
(74) Representative: Katérle, Axel
(86) International application number: PCT/US2012/031922
(87) International publication number: WO 2012/135870

(56) References cited:
- EP-A2- 0 809 973
- US-A- 5 545 164
- US-A- 5 743 907
- US-A- 5 814 046
- US-B1- 6 280 443
- US-B2- 7 314 467

## Description

### Technical Field

The present disclosure relates to devices and methods for stabilizing posterior elements of the spine. More particularly, the disclosure relates to a posterior cervical stabilization system that may secure to associated occipital C1 C2 and thoracic spine.

### Background

The vertebrae in a patient's spinal column are linked to one another by the disc and the facet joints. The facet joints control movement of the vertebrae relative to one another. Each vertebra has a pair of articulating surfaces located on the left side, and a pair of articulating surfaces located on the right side, and each pair includes a superior articular surface, which faces upward, and an inferior articular surface, which faces downward. Together the superior and inferior articular surfaces of adjacent vertebra form a facet joint. Facet joints are synovial joints, which means that each joint is surrounded by a capsule of connective tissue and produces a fluid to nourish and lubricate the joint. The joint surfaces are coated with cartilage allowing the joints to move or articulate relative to one another.

Facet joints and/or discs that become diseased, degenerated, impaired, or otherwise painful can require surgery to restore function to the three joint complex. Traditionally, diseased levels in the spine were fused to one another. While such a technique may relieve pain, the fusing effectively prevents motion between at least two vertebrae. As a result of the limited motion, additional stress may be applied to the adjoining levels, thereby potentially leading to further damage to the spine.

More recently, techniques have been developed to restore normal function to the facet joints. One such technique involves covering the facet joint with a cap to preserve the bony and articular structure. Capping techniques, however, are limited in use as they will not remove the source of the pain in osteoarthritic joints. Caps are also disadvantageous as they must be available in a variety of sizes and shapes to accommodate the wide variability in the anatomical morphology of the facets. Caps also have a tendency to loosen over time, potentially resulting in additional damage to the joint and/or the bone support structure containing the cap.

Another conventional technique for restoring the normal function to the posterior element of the spine involves arch replacement, in which superior and inferior prosthetic arches are implanted to extend across the vertebra typically between the spinous process. These arches can articulate relative to one another to replace the articulating function of the facet joints. One drawback of current articulating facet replacement devices, however, is that they require the facet joints to be resected. Moreover, alignment of the articulating surfaces with one another can be challenging.

EP 0 809 973 A discloses another apparatus for retaining first and second cervical vertebrae of a spinal column in a desired spatial relationship, which comprises two plates, two rods and fastener means for connecting the respective plate to the respective cervical vertebra.

Accordingly, it may be desirable to provide improved systems for stabilizing posterior elements of the spine that capable of mimicking the natural function of the facet joints.

### Summary of Invention

The above object is achieved with the features of independent claim 1. According to various aspects of the disclosure, a posterior cervical stabilization system may be secured to associated occipital C₁ C₂ and thoracic spine. The system may be modular such that it can be attached to the skull or, using fluorescent imaging, can be placed at C₁ C₂. The system may include modules configured for use primarily with the lateral masses of the cervical spine C₃-C₆ or, with the secured variation, for use with the C₇ pedicles or the thoracic pedicles. Thus, the system could be used from the skull through the Tspine.

The C₃-C₆ lateral mass modules would contain a pre-drilled hole with orientation of about 20-40° laterally (away from the spinal cord) and rostrally about 20-40° (toward the head). The module can be held by a fork device (e.g., top-loading) that can secure the position of the module in conjunction with tiny spikes on the bottom of the modules prior to holes being drilled in the bone, for example, with top loading nuts. The lateral mass modules could then be secured to occipital C₁, C₂, C₇, thoracic with screws. The C₇ or thoracic modules are oriented with pre-drilled holes about 10-15° medially.

Some further advantages and embodiments may become evident from the attached drawings.

### Brief Description of the Drawings

FIG. 1 is a top view of an exemplary posterior cervical stabilization system in accordance with various aspects of the disclosure.
FIG. 2 is a bottom view of an exemplary posterior cervical stabilization system in accordance with various aspects of the disclosure.
FIG. 3 is a lateral view of an exemplary posterior cervical stabilization system in accordance with various aspects of the disclosure.
FIG. 4 is a top view of an exemplary posterior cervical stabilization system in accordance with various aspects of the disclosure.
FIGS. 5-7 are alternate view of the exemplary system of FIG. 2.
FIG. 8 is top view of another exemplary posterior cervical stabilization system in accordance with various aspects of the disclosure.

### Detailed Description

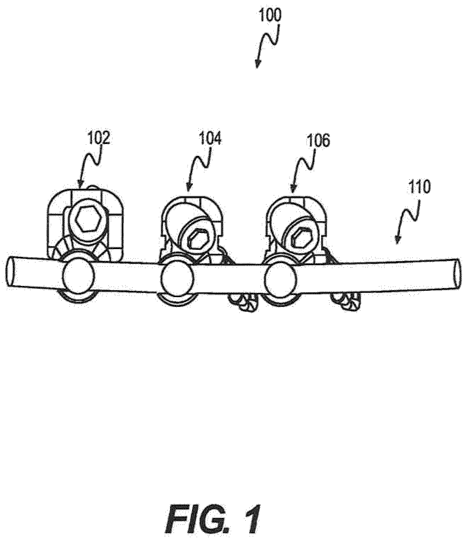

FIG. 1 illustrates an exemplary posterior cervical stabilization system in accordance with the disclosure. A posterior cervical stabilization system 100 may include two or more modular components 102, 104, 106 with a top loading rod 110. Although the illustrated embodiments show systems 100 have two and three modules, it should be appreciated that the systems contemplated by this disclosure may include any number of modules.

The modules 102, 104, 106 are oriented on the lateral masses with fixation with titanium screws into the bone of the lateral mass with an orientation outward about 20-40° and angulation rostrally about 20-40°. The modules 102, 104, 106 have a low profile and either lock to the interphase of the module or are held with a locking mechanism. The laterally held rod 110 can be polyaxial or non-polyaxial with its connection to the modules with a top loading nut.

As shown in FIG. 1, module 102 may be oriented medially about 10-15° and used with a C₇ or thoracic screw. Modules 104, 106 are the lateral mass components oriented laterally about 20-40° and rostrally about 20-40°.

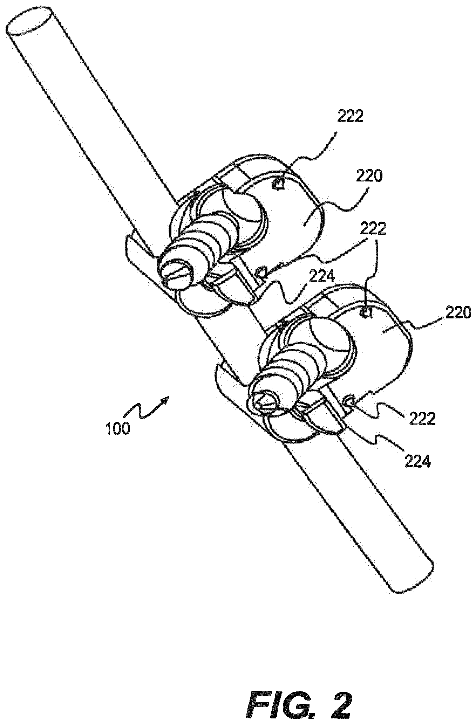

Referring to FIG. 2, a bottom surface 220 of the modules 104, 106 may include one or more small spikes 222, for example, 1-2 mm extensions from the bottom surface. The spikes 222 can be used to temporarily hold the modules to a lateral mass bone prior to the creation of screw holes for receiving the screws. The bottom surface 220 of the modules 104, 106 may also include a lateral flange 224 oriented toward the lateral aspect of the lateral mass to help with centering the module 104, 106. Although not depicted in FIG. 2, module 102 may similarly include spikes and a lateral flange for holding and centering the module during a procedure.

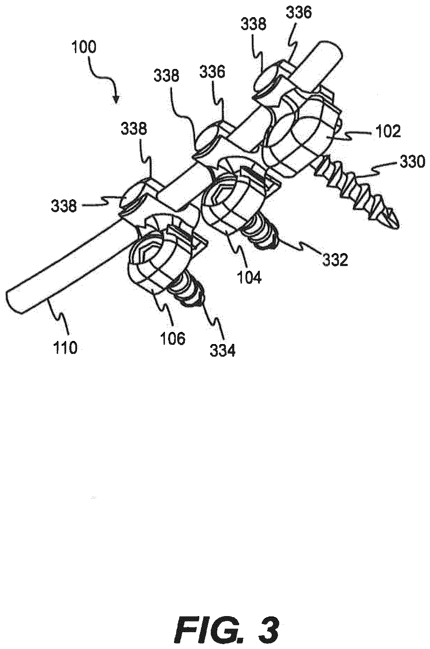

Referring now to FIG. 3, the module 102 includes the C7 or thoracic screw 330 oriented about 10-15° medially. The modules 104, 106 include the lateral mass screws 332, 334, which are oriented about 20-40° laterally and about 20-40° rostrally away from the spinal cord.

The modules 102, 104, 106 are individually oriented over the center of the lateral mass, and each include a predrilled hole with appropriate lateral and rostral orientation. Accordingly, the modules 102, 104, 106 can be held and holes can be drilled, tapped, and the screws fixated and locked to the modules.

The rod 110 may be formed of titanium, polyethylene ketone (PEEK), carbon fiber, or other suitable composites that provide the desired characteristics of flexibility/stiffness, biocompatibility, imaging characteristic, and the like. The modules 102, 104, 106 include a top-loading polyaxial or non-polyaxial screw top 336 that receives the rod 110. The rod 110 can be secured to the modules with top loading nuts 338 that couple with the screw tops 336. It should be appreciated that other conventional securing mechanisms are contemplated by this disclsoure

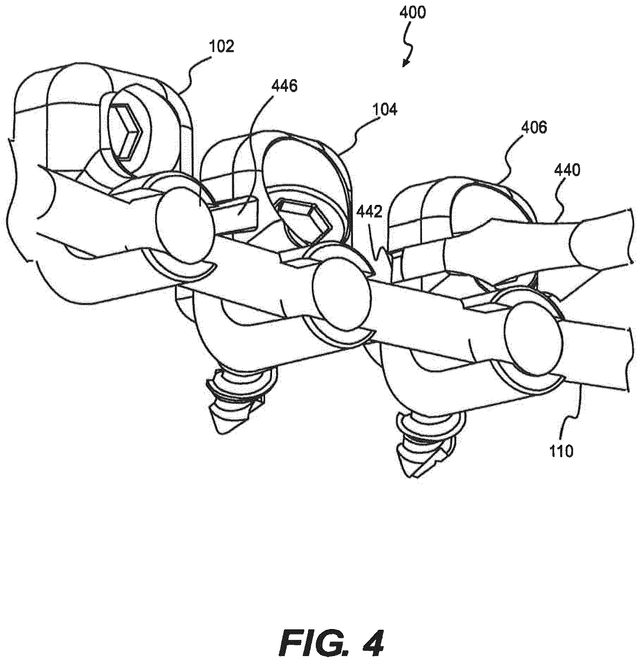

FIG. 4 illustrates the stabilization system 400 during an exemplary implantation procedure. The system 400 includes a top-loading fork device 440 that can straddle a module 406 and couple with the module via engagement with grooves 442 in the sides of the module 406. The fork device 440 can thus temporarily secure the positioning of the module during drilling and placement of the screws.

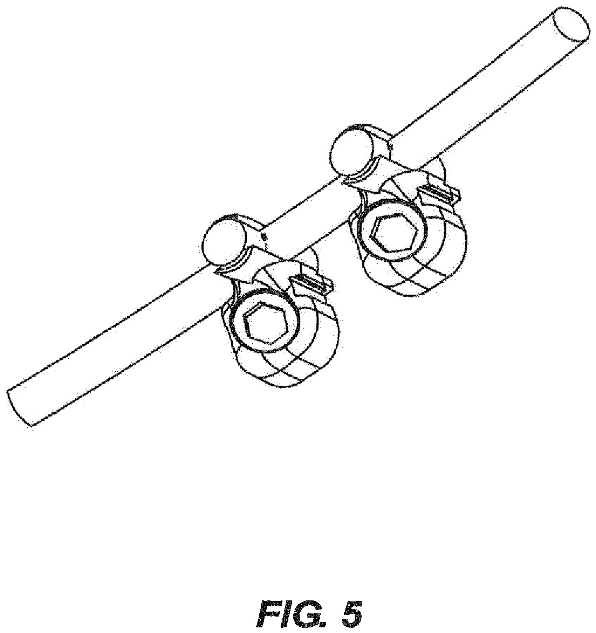

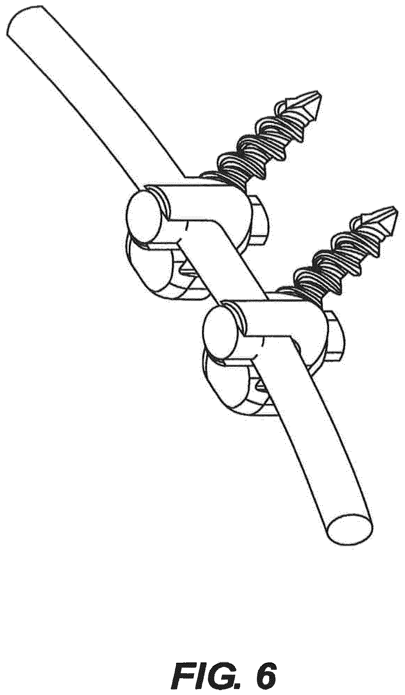

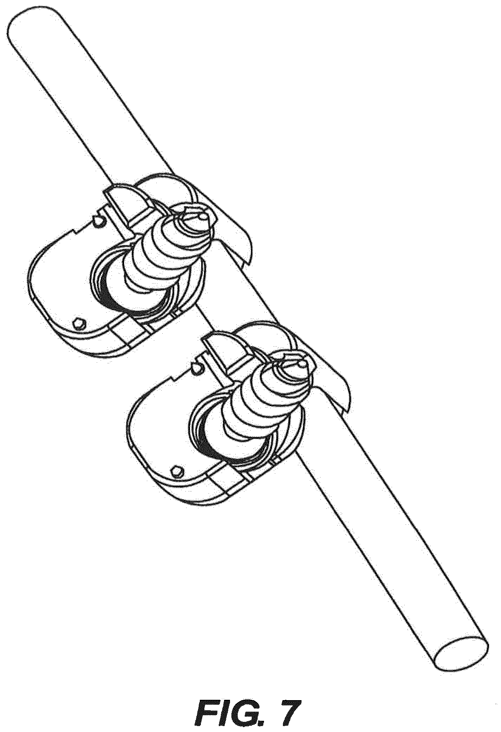

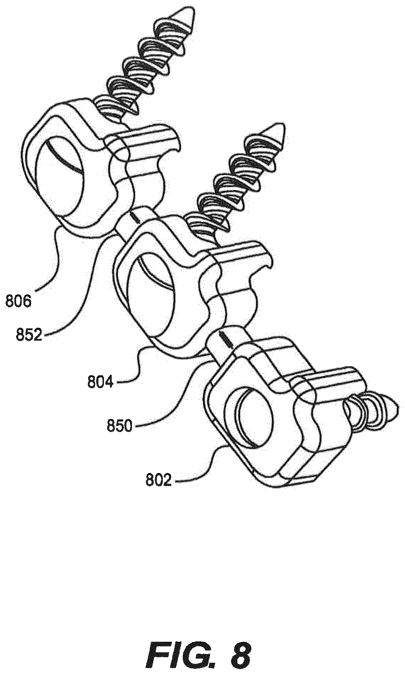

FIGS. 5-7 illustrate different view of the system shown in FIG. 2 so that additional aspects and features of the disclosure can be appreciated. FIG. 8 illustrates an alternative stabilization system 800 where the modules 802, 804, 806 are fixedly coupled to one another via connecting elements 850, 852 rather than via a rod.

Posterior cervical stabilization systems according to the present disclosure are better than other conventional systems because the present posterior cervical stabilization systems are able to produce more reproduceable screw angles, which promotes more safety.

## Claims

1. An implantable posterior cervical stabilization system (100, 400, 800) comprising a lateral mass component (104, 106), the lateral mass component (104, 106) comprising:
a first end, a second end being opposed to the first end, and a bottom surface (220);
a lateral flange (224) extending from the bottom surface (220) adjacent the first end, the lateral flange (224) being disposable to orientate toward a lateral mass of a vertebra for centering the lateral mass component (104, 106), the lateral flange (224) having a first height;
an extension (222) extending from the bottom surface (220), the extension (222) having a second height less than the first height; and
a predrilled hole extending through the lateral mass component from the bottom surface (220), the predrilled hole being angled toward the lateral flange (224), wherein the predrilled hole is angled at an angle within the range of about 20-40 degrees in a lateral direction.

2. The implantable posterior cervical stabilization system (100, 400, 800) of claim 1,
wherein the predrilled hole is angled at an angle within the range of about 20-40 degrees in the rostral direction.

3. The implantable posterior cervical stabilization system (100, 400, 800) of claim 1,
wherein the bottom surface (220) comprises a flat surface.

4. The implantable posterior cervical stabilization system (100, 400, 800) of claim 1,
wherein the lateral flange (224) is disposed proximate only a portion of the first end.

5. The implantable posterior cervical stabilization system (100, 400, 800) of claim 4,
wherein the lateral flange (224) is disposed in a corner of the bottom surface (220).

6. The implantable posterior cervical stabilization system (100, 400) of claim 1, wherein the bottom surface (220) comprises a rod receiving portion (336) sized to receive a fixation rod (110), the rod receiving portion (336) being located along a lateral portion of the bottom surface (220).

7. The implantable posterior cervical stabilization system (100, 400) of claim 6, wherein the rod receiving portion (336) is integrally formed with the lateral mass component (104, 106).

8. The implantable posterior cervical stabilization system (100, 400) of claim 6, wherein the rod receiving portion (336) comprises a polyaxial portion configured to receive a rod (110).

9. The implantable posterior cervical stabilization system (100, 400) of claim 6, comprising a rod (110) connected to the rod receiving portion (336).

10. The implantable posterior cervical stabilization system (100, 400) of claim 9, comprising a fastener (332, 334) which extends through the predrilled hole beyond the lateral mass component (104, 106) so as to penetrate the lateral mass of the vertebra.

11. The implantable posterior cervical stabilization system (100, 400) of claim 10, wherein the fastener (332, 334) has a length sufficient to extend below the rod (110) when viewed from a top view.

12. The implantable posterior cervical stabilization system (100, 400) of claim 6, comprising a top loading nut (338) configured to interface with the rod receiving portion (336) to secure a rod (110) in the rod receiving portion (336).

13. The implantable posterior cervical stabilization system (100, 400, 800) of claim 1,
wherein the predrilled hole is oriented in the lateral direction thereby directing a fastener (332, 334) into a lateral mass of a patient.

14. The implantable posterior cervical stabilization system (100, 400, 800) of claim 1,
wherein the extension (222) comprises a spike having a height in the range of 1-2mm.

## Patentansprüche

1. Implantierbares posteriores Halswirbelstabilisierungssystem (100, 400, 800) mit einer lateralen Massenkomponente (104, 106), wobei die laterale Massenkomponente (104, 106) umfasst:
ein erstes Ende, ein dem ersten Ende gegenüberliegendes zweites Ende und eine Bodenfläche (220);
einen seitlichen Flansch (224), der sich von der dem ersten Ende benachbarten Bodenfläche (220) erstreckt, wobei der seitliche Flansch (224) zur Ausrichtung in Richtung einer lateralen Masse eines Wirbels zur Zentrierung der lateralen Massenkomponente (104, 106) anordenbar ist, und wobei der seitliche Flansch (224) eine erste Höhe aufweist;
einen Fortsatz (222), der sich von der Bodenfläche (220) erstreckt, wobei der Fortsatz (222) eine zweite Höhe aufweist, die kleiner ist als die erste Höhe; und
eine Bohrung, die sich von der Bodenfläche (220) durch die laterale Massenkomponente erstreckt, wobei die Bohrung zum seitlichen Flansch (224) hin abgewinkelt ist, wobei die Bohrung in einem Winkel im Bereich von etwa 20-40 Grad in einer lateralen Richtung abgewinkelt ist.

2. Implantierbares posteriores Halswirbelstabilisierungssystem (100, 400, 800) nach Anspruch 1,
wobei die Bohrung unter einem Winkel im Bereich von etwa 20-40 Grad in Rostralrichtung abgewinkelt ist.

3. Implantierbares posteriores Halswirbelstabilisierungssystem (100, 400, 800) nach Anspruch 1,
wobei die Bodenfläche (220) eine ebene Fläche aufweist.

4. Implantierbares posteriores Halswirbelstabilisierungssystem (100, 400, 800) nach Anspruch 1,
wobei der seitliche Flansch (224) in der Nähe nur eines Abschnitts des ersten Endes angeordnet ist.

5. Implantierbares posteriores Halswirbelstabilisierungssystem (100, 400, 800) nach Anspruch 4,
wobei der seitliche Flansch (224) in einer Ecke der Bodenfläche (220) angeordnet ist.

6. Implantierbares posteriores Halswirbelstabilisierungssystem (100, 400) nach Anspruch 1,
wobei die Bodenfläche (220) einen Stabaufnahmeabschnitt (336) aufweist, der so bemessen ist, dass er einen Fixierstab (110) aufnehmen kann, wobei der Stabaufnahmeabschnitt (336) entlang eines seitlichen Abschnitts der Bodenfläche (220) gelegen ist.

7. Implantierbares posteriores Halswirbelstabilisierungssystem (100, 400) nach Anspruch 6,
wobei der Stabaufnahmeabschnitt (336) einstückig mit der lateralen Massenkomponente (104, 106) gebildet ist.

8. Implantierbare posteriores Halswirbelstabilisierungssystem (100, 400) nach Anspruch 6,
wobei der Stabaufnahmeabschnitt (336) einen polyaxialen Abschnitt umfasst, der dazu ausgebildet ist, einen Stab (110) aufzunehmen.

9. Implantierbares posteriores Halswirbelstabilisierungssystem (100, 400) nach Anspruch 6,
das eine mit dem Stabaufnahmeabschnitt (336) verbundene Stange (110) aufweist.

10. Implantierbares posteriores Halswirbelstabilisierungssystem (100, 400) nach Anspruch 9,
das ein Befestigungselement (332, 334) aufweist, das sich durch die Bohrung über die laterale Massenkomponente (104, 106) hinaus erstreckt, um die laterale Masse des Wirbels zu durchdringen.

11. Implantierbares posteriores Halswirbelstabilisierungssystem (100, 400) nach Anspruch 10,
wobei das Befestigungselement (332, 334) eine Länge aufweist, die ausreicht, um sich bis unterhalb des Stabs (110) zu erstrecken, bei einer Betrachtung von oben.

12. Implantierbares posteriores Halswirbelstabilisierungssystem (100, 400) nach Anspruch 6,
mit einer Toplader-Mutter (338), die dazu ausgebildet ist, mit dem Stabaufnahmeabschnitt (336) gekoppelt zu werden, um einen Stab (110) in dem Stabaufnahmeabschnitt (336) zu sichern.

13. Implantierbares posteriores Halswirbelstabilisierungssystem (100, 400, 800) nach Anspruch 1,
wobei die Bohrung in der lateralen Richtung ausgerichtet ist, um so ein Befestigungselement (332, 334) in eine laterale Masse eines Patienten zu führen.

14. Implantierbares posteriores Halswirbelstabilisierungssystem (100, 400, 800) nach Anspruch 1,
wobei der Fortsatz (222) einen Dorn mit einer Höhe im Bereich von 1-2 mm aufweist.

## Revendications

1. Système de stabilisation de cervicale postérieure implantable (100, 400, 800) comprenant un composant de masse latérale (104, 106), le composant de masse latérale (104, 106) comprenant :
une première extrémité, une seconde extrémité en regard de la première extrémité, et une surface inférieure (220) ;
un rebord latéral (224) partant de la surface inférieure (220) adjacente à la première extrémité, le rebord latéral (224) pouvant être positionné pour s'orienter dans la direction d'une masse latérale d'une vertèbre pour centrer le composant de masse latérale (104, 106), le rebord latéral (224) ayant une première hauteur ;
une extension (222) partant de la surface inférieure (220), l'extension (222) ayant une seconde hauteur inférieure à la première hauteur ; et
un trou pré-percé s'étendant à travers le composant de masse latérale à partir de la surface inférieure (220), le trou pré-percé étant incliné en direction du rebord latéral (224), le trou pré-percé étant incliné selon un angle dans la plage comprise environ entre 20 et 40 degrés dans un sens latéral.

2. Système de stabilisation de cervicale postérieure implantable (100, 400, 800) selon la revendication 1, le trou pré-percé étant incliné dans la plage comprise environ entre 20 et 40 degrés dans le sens rostral.

3. Système de stabilisation de cervicale postérieure implantable (100, 400, 800) selon la revendication 1, la surface inférieure (220) comprenant une surface plate.

4. Système de stabilisation de cervicale postérieure implantable (100, 400, 800) selon la revendication 1, le rebord latéral (224) étant disposé à proximité uniquement d'une partie de la première extrémité.

5. Système de stabilisation de cervicale postérieure implantable (100, 400, 800) selon la revendication 4, le rebord latéral (224) étant disposé dans un coin de la surface inférieure (220).

6. Système de stabilisation de cervicale postérieure implantable (100, 400) selon la revendication 1, la surface inférieure (220) comprenant une partie de réception de tige (336) dimensionnée pour recevoir une tige de fixation (110), la partie de réception de tige (336) étant située le long d'une partie latérale de la surface inférieure (220).

7. Système de stabilisation de cervicale postérieure implantable (100, 400) selon la revendication 6, la partie de réception de tige (336) faisant partie intégrante du composant de masse latérale (104, 106).

8. Système de stabilisation de cervicale postérieure implantable (100, 400) selon la revendication 6, la partie de réception de tige (336) comprenant une partie polyaxiale conçue pour recevoir une tige (110).

9. Système de stabilisation de cervicale postérieure implantable (100, 400) selon la revendication 6, comprenant une tige (110) reliée à la partie de réception de tige (336).

10. Système de stabilisation de cervicale postérieure implantable (100, 400) selon la revendication 9, comprenant un élément de fixation (332, 334) qui s'étend à travers le trou pré-percé au-delà du composant de masse latérale (104, 106) afin de pénétrer dans la masse latérale de la vertèbre.

11. Système de stabilisation de cervicale postérieure implantable (100, 400) selon la revendication 10, l'élément de fixation (332, 334) ayant une longueur suffisante pour s'étendre sous la tige, lorsqu'il est observé du dessus.

12. Système de stabilisation de cervicale postérieure implantable (100, 400) selon la revendication 6, comprenant un écrou de charge supérieur (338) conçu pour relier la partie de réception de tige (336) afin de fixer une tige (110) dans la partie de réception de tige (336).

13. Système de stabilisation de cervicale postérieure implantable (100, 400, 800) selon la revendication 1, le trou pré-percé étant orienté dans la direction latérale permettant ainsi de guider un élément de fixation (332, 334) dans une masse latérale d'un patient.

14. Système de stabilisation de cervicale postérieure implantable (100, 400, 800) selon la revendication 1, l'extension (222) comprenant une pointe ayant une hauteur dans la plage comprise entre 1 et 2 mm.
